# EUROPEAN PATENT SPECIFICATION

(11) **EP 4 525 791 B1**
(45) Date of publication and mention of the grant of the patent: **01.07.2026**
(21) Application number: 23723239.2
(22) Date of filing: 03.05.2023
(51) Int. Cl.: A61F 5/01

(54) **ANKLE-FOOT ORTHOPAEDIC DEVICE**
ORTHOPÄDISCHE VORRICHTUNG FÜR KNÖCHEL UND FUSS
DISPOSITIF ORTHOPÉDIQUE CHEVILLE-PIED

(30) Priority: 16.05.2022 GB 202207094
(43) Date of publication of application: 26.03.2025
(73) Proprietor: Orthoped Ltd, Hayling Island Hampshire PO11 9LG (GB)
(72) Inventor: NARBOROUGH, Claire, Hayling Island Hampshire PO11 9LG (GB)
(74) Representative: Sirius IP
(86) International application number: PCT/GB2023/051163
(87) International publication number: WO 2023/222991

(56) References cited:
- WO-A1-2020/004816
- US-A- 4 651 723
- US-A1- 2004 043 879
- US-A1- 2008 171 956
- US-A1- 2013 138 030
- US-A1- 2021 393 428
- ANONYMOUS: "Adjustable Drop Foot Brace for Walking, AFO Brace Foot Drop Orthosis Universal Size", OBERHEALTH, 14 May 2021 (2021-05-14), pages 1 - 3, XP093050938, Retrieved from the Internet <URL:https://www.oberhealth.com/product/drop-foot-brace-for-walking-afo-brace-foot-drop-orthosis> [retrieved on 20230601]

## Description

The present invention relates to an ankle-foot orthopaedic device. In particular, the present invention relates to an ankle-foot orthopaedic device for children. The present invention also relates to a method for installing the ankle-foot orthopaedic device and a method for manufacturing the ankle-foot orthopaedic device.

### BACKGROUND OF INVENTION

In the UK, 46,720 children have a condition known as footdrop. Footdrop is a physiological disorder resulting from muscle weakness or paralysis which creates difficulty dorsiflexing (such as lifting the front of the foot). This becomes particularly problematic during the gait cycle and can cause frequent trips and falls. Clinical observation suggests that approximately 25% of the children diagnosed with footdrop will have permanent footdrop of one or both legs.

Early intervention for children effected by footdrop has been shown to improve physical, emotional and social outcomes for the children and their families. As a result, this can reduce the associated burden from the condition on society and to allow the children to develop and lead independent lives.

Conventional devices for the treatment of footdrop include rigid splints for severe cases of footdrop or dynamic orthosis (e.g. silicone or lycra socks) for milder cases of footdrop.

In order to obtain a splint, the user must attend a minimum of two appointments for measuring/casting and fitting. Children grow and as such the splints may need to be replaced regularly, for example three times a year.

Splints can hinder independent mobility and can therefore be problematic for children to engage with physical education and sports. Furthermore, splints can be bulky resulting in a user having to purchase replacement, larger size shoes, in order for the user to be able to wear shoes whilst wearing the splint.

Many cases of footdrop also affects one arm of the user which can effect the ability of the user to independently put on their splints by themselves. This can mean that the user will require additional support and assistance at home and at school.

US2013/138030 discloses an orthotic for alleviating foot drop, the orthotic providing resilient support between the leg of a user and a foot covering, the foot covering having a dorsal portion, comprising: an elongate elastic strap having first and second end portions; an ankle brace removably engageable with the leg of the user, the ankle brace including a primary strap loop for shiftably receiving said elongate elastic strap there through; and first and second attachment elements fixedly carried by respective ones of said first and second end portions of said elongate elastic strap for detachably coupling said elongate elastic strap to said foot covering dorsal portion.

US2008/171956 discloses an apparatus that can be used to reduce a foot drop condition, the apparatus comprising: a lower leg member having an opening, wherein the lower leg member has a size and dimension to be coupled to a portion of a lower leg below a calf muscle and above a foot, the lower leg member having an inner layer of a first material and an outer layer of a second material; an elongate closure member disposed on the lower leg member and configured to releasably secure the lower leg member to the portion of the lower leg; and a pair of attachment members disposed below the elongate closure member and located on opposing sides of the opening of the lower member, wherein the pair of attachment members can be releasably coupled to a lacing member that is coupled to footwear on the foot and the pair of attachment members can be used to support a force to orient the foot at a desired orientation when the lacing member is secured.

US2021/393428 discloses a device comprising: a support configured to wrap around at least a portion of a lower leg of a user; a lace support attached to the support and configured to receive a lace; and a reel-based fastener attached to the support, wherein the reel-based fastener comprises a reel for receiving the lace and adjusting a tautness of the lace.

WO2020/004816 discloses a foot drop support structure enabling natural walking in a state in which the support structure keeps lifting the foot which drops when walking. The structure is worn on a foot area of a foot drop patient showing a foot dropping symptom occurring when a nerve damage, muscle abnormality, muscular strength decrease, peroneal nerve palsy, etc. occur due to a stroke, hemiplegia, cerebral palsy, a bone fracture, an accident, etc There is therefore a need for a device to support a user with footdrop which is easier to put on so as to encourage independence. There is also for a need for a device which is less restrictive and cumbersome to enable the user to freely participate in sports. There is a need for an adjustable device which can accommodate a range of user's lower limb dimensions and can also accommodate for growth.

### STATEMENT OF INVENTION

In a first aspect of the present invention, there is provided an ankle-foot orthopaedic device for the treatment of foot drop, the device comprising:
an openable lower leg attachment portion configured in use to be positioned against and to extend around a portion of a lower leg of a user, in which a first end of the lower leg attachment portion is engageable to a portion of the lower leg attachment portion at or adjacent a second opposed end thereof, and
a second attachment portion comprising an elongate member having a first end located at or adjacent the openable lower leg attachment portion, and a second opposed end comprising a shoe attachment member configured in use for direct engagement with a fastening of a shoe of a user,
in which the second opposed end of the openable lower leg attachment portion provides an aperture configured to enable the first end to pass therethrough, and in which the first end of the lower leg attachment portion comprises at least one attachment member for securing the first end in position relative to the second opposed end of the lower leg attachment portion.

The fastening of a shoe may be any closure mechanism for closing the shoe across a foot of a user, such as for example a lace and/or hook and loop fasteners (such as for example Velcro^{®}).

The lower leg attachment portion is preferably moveable between:
a first open position in which the first end of the lower leg attachment portion is disengaged from, and preferably moveable relative to, the second opposed end thereof; and
a second closed position in which a portion of the lower leg attachment portion adjacent the first end thereof extends through the aperture at the second end, and the at least one attachment member secures the first end adjacent an outer surface of the lower leg attachment portion.

In one embodiment, in the first open position, the first end of the lower leg attachment portion is spaced apart and separated from the second opposed end thereof.

In one embodiment, in the first open position, the first end of the lower leg attachment portion extends through the aperture at the second end thereof, and is disengaged from and moveable relative to the second end.

In one embodiment, in the first open position, first end of the lower leg attachment portion extends through the aperture at the second end thereof to form a loop, and in which the first end is disengaged from and moveable in a circumferential direction of the loop relative to the second end of the lower leg attachment portion.

According to a second aspect, the present invention provides a method of single-handed installation of an ankle-foot orthopaedic device as herein described to the leg and shoe of a user;
positioning an openable lower leg attachment portion to extend around a portion of a lower leg of a user;
optionally passing a first end of the lower leg attachment portion through the aperture provided at a second end thereof is engageable to a second end thereof;
adjusting the position of the first end relative to the second end of the lower leg attachment portion to position the lower leg attachment portion adjacent the lower leg of a user;
securing the first end to a portion of the lower leg attachment portion;
engaging the shoe attachment member with a fastener of a shoe of a user.

In one embodiment, the first end of the lower leg attachment portion is secured at or adjacent a second end thereof.

According to a third aspect of the present invention, there is provided a method for manufacturing an ankle-foot orthopaedic device as herein described, the method comprising:
obtaining an openable lower leg attachment portion configured in use to be positioned against and to extend around a portion of a lower leg of a user, in which a first end of the lower leg attachment portion is engageable to a portion of the lower leg attachment portion, preferably at or adjacent a second opposed end thereof; and
engaging a first end of a second attachment portion comprising an elongate member at or adjacent the openable lower leg attachment portion.

The ankle-foot orthopaedic device is preferably a child's ankle-foot orthopaedic device. It is however to be understood that the ankle-foot orthopaedic device may be used by any suitable user and is not to be considered to be limited for use by a child.

The ankle-orthopaedic device is preferably able to be put on by a single hand of a user. As a result, the ankle-orthopaedic device may be able to be used by young children who may also suffer from weakness in one arm. Furthermore, the ankle-orthopaedic device of the present invention therefore enables the user to put the splint on independently without requiring assistance from others. The ankle-orthopaedic device of the present invention therefore encourages the user to develop an independent life without impacting on the physical, emotional and social aspects of the user's life.

The ankle-foot orthopaedic device is preferably free from contact with a foot or inner portion of a shoe of a user. As a result, the device of the present invention does not have any engagement with the inside of a user's shoe. The user may therefore wear the device without the device creating an obstruction preventing the user's normal shoes to be worn.

The shoe attachment member is preferably releasably engageable to the fastener of a shoe of a user. The shoe attachment member is preferably releasably engageable to the fastener of a shoe of a user from the outside of a shoe of a user. As such, the user may easily and effectively change shoes as required without having to dismantle the device or device/shoe combination. In contrast, the user may simply disengage the shoe attachment member from the fastener of the first shoe and re-engage the shoe attachment member with a fastener of a second shoe. The ankle-foot orthopaedic device of the present invention therefore enables the user to have a greater degree of freedom with regards to changing footwear.

The lower leg attachment portion is preferably composed of flexible material. The lower leg attachment portion is preferably composed of neoprene. The lower leg attachment portion preferably comprises an elasticated portion.

The lower leg attachment portion is preferably an elongate member. For example, an elongate member defining a longitudinal axis extending between the first and second ends thereof.

The lower leg attachment portion preferably comprises an inner surface configured in use to be positioned adjacent a lower leg of a user and an opposed outer surface.

The outer surface preferably provides the at least one attachment member.

The lower leg attachment portion preferably comprises at least one pair of cooperative attachment members. In one embodiment, a first attachment member is located on the outer surface of the lower leg attachment portion at or adjacent the first end thereof, and a cooperative second attachment member located on the outer surface of the lower leg attachment portion and spaced apart from the first attachment member (for example between the first and second ends of the lower leg attachment portion).

The at least one attachment member may comprise any suitable attachment members such as for example buttons, toggles, studs, poppers, zips, or any combination thereof. The at least one attachment member preferably comprises hook and loop fasteners.

The aperture provided at the second end of the lower leg attachment portion may for example be an elongate slit. The aperture, for example elongate slit, may define a longitudinal axis which extends substantially perpendicular to the longitudinal axis defined between the first and second ends of the lower leg attachment potion.

According to the invention, the lower leg attachment portion is be T-shaped. The first end of the lower leg attachment portion has a smaller width (when measured between opposed side portions thereof) than the second end of the lower leg attachment portion. The aperture, such as for example an elongate slit, provided at or adjacent the second end of the lower leg attachment portion, is sized to enable to receive the full width of the first end of the lower leg attachment portion.

The shoe attachment member preferably comprises a hooked portion. In one embodiment, the shoe attachment member comprises a plurality of hooked portions. The (or each) hooked portion comprises a first end secured to the shoe attachment portion, and a second end defining a hooked end defining a recess to receive and engage a fastener of a shoe of a user.

The elongate member is preferably a tubular member, such as for example a rope.

In one embodiment, the ankle-foot orthopaedic device further comprises an adjustment mechanism configured in use to adjust the separation of the shoe attachment member at the second opposed end of the second attachment portion from the lower leg attachment portion. The adjustment mechanism is preferably located at or adjacent the first end of the second attachment portion.

The adjustment mechanism may comprise a toggle mechanism in communication with the elongate member (for example, the elongate member extends through the toggle mechanism) to control the extension of the second opposed end of the elongate member from the lower leg attachment portion.

In one embodiment, the second attachment portion is configured in use to extend around at least a portion of the lower leg attachment portion. The second attachment portion is preferably configured in use to encompass or to extend entirely around the lower leg attachment portion.

The lower leg attachment portion may further comprise at least one guide member defining an open-ended channel configured in use to receive at least a portion of the second attachment portion therein and to enable movement of the second attachment portion through the channel. In one embodiment, the at least one guide member is provided on an outer surface of the lower leg attachment portion. A pair of guide members may be provided on opposing sides of the lower leg attachment portion.

### BRIEF DESCRIPTION

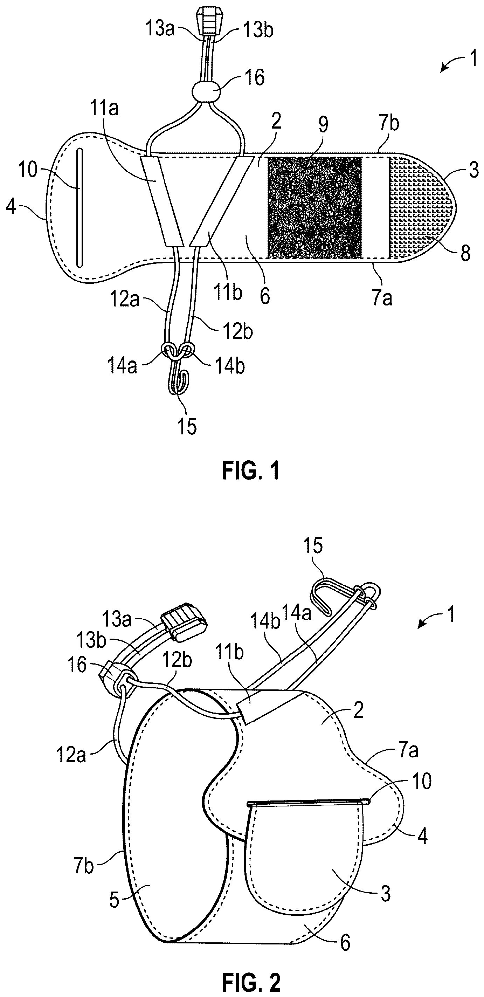

Figure 1 is a schematic illustration of the ankle-foot orthopaedic device in the first open position according to one embodiment of the present invention;
Figure 2 is a schematic illustration of the ankle-foot orthopaedic device in the second closed position according to Figure 1;

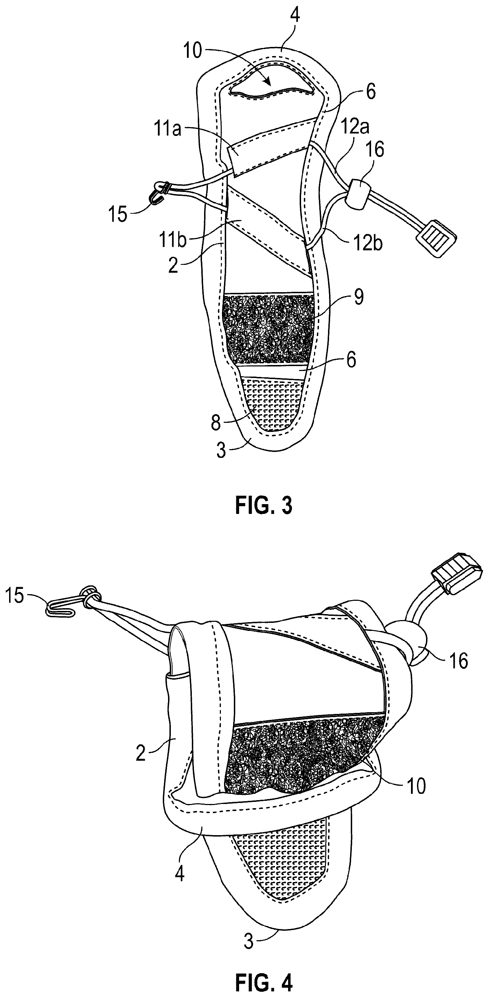

Figure 3 is a photograph of the ankle-foot orthopaedic device in the first open position according to a further embodiment of the present invention;
Figure 4 is a photograph of the ankle-foot orthopaedic device in the second closed position according to Figure 3;

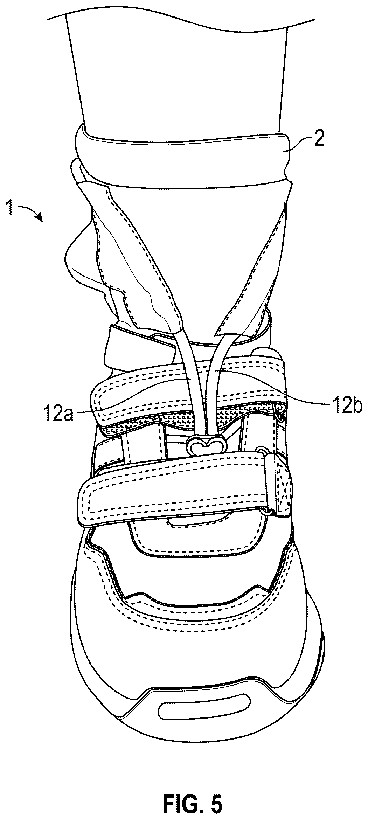

Figure 5 is a photograph of the front view of the ankle-foot orthopaedic device of Figure 3 being worn in the second closed position;

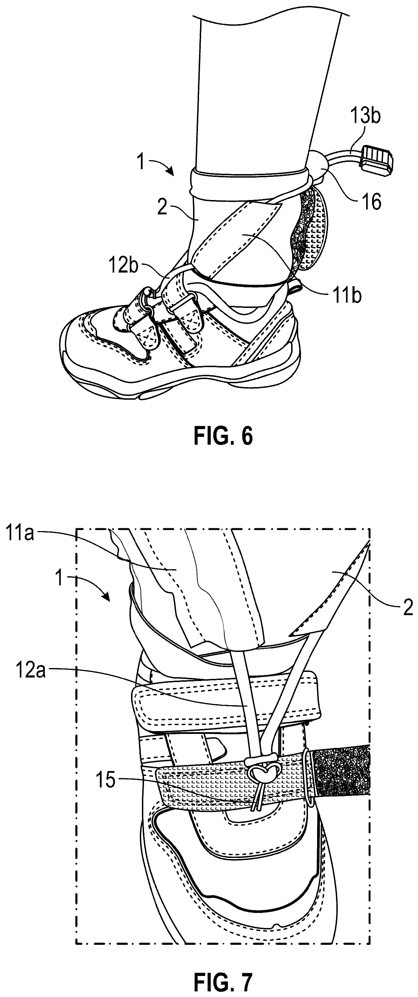

Figure 6 is a photograph of the side view ankle-foot orthopaedic device of Figure 3 being worn in the second closed position; and
Figure 7 is a photograph of the front view ankle-foot orthopaedic device being worn in the second closed position during securement to the shoe of the user.

### DETAILED DESCRIPTION

With reference to the Figures, the ankle-foot orthopaedic device 1 for the treatment of foot drop comprises an openable lower leg attachment portion 2 configured in use to be positioned against and to extend around a portion of a lower leg of a user.

The openable lower leg attachment portion 2 is composed of neoprene.

The lower leg attachment portion 2 is an elongate member comprising a first end 3, opposed second end 4, inner surface 5 configured in use to be positioned adjacent a lower leg of a user and an opposed outer surface 6. A longitudinal axis is defined between the first and second ends 3, 4 of the lower leg attachment portion 2.

The first end 3 has a width (as measured between side portions 7a, 7b) which is less than the width of the second end 4 of the lower leg attachment portion 2.

The outer surface 6 comprises a first attachment member 8 in the form of hook and loop fasteners located at the first end 3 of the lower leg attachment portion 2. The outer surface 6 further comprises a second attachment member 9 in the form of hook and loop fasteners spaced apart from the first attachment member 8 and located between the first 3 and second 4 ends of the lower leg attachment portion 2.

The second end 4 provides an aperture 10 in the form of an elongate slit extending in a direction between opposed side portions 7a, 7b of the lower leg attachment portion 2. The longitudinal axis of the aperture 10 extends substantially perpendicular to the longitudinal axis defined between the first and second ends 3, 4 of the lower leg attachment portion 2. The aperture 10 extends between the inner and outer surfaces 5, 6 of the lower leg attachment portions.

The aperture 10 has a length slightly larger than the width of the first end 3 such that the first end 3 may be received through the aperture 10 without any additional folding or bending.

The outer surface 6 of the lower leg attachment portion 2 further comprises a pair of spaced apart guide portions 11a, 11b. Each guide portion is an open ended channel.

The device 1 further comprises two second attachment portions 12a, 12b formed from a single, elongate member. Each second attachment portion 12a, 12b is configured in use to extend around an opposed side of a lower leg of a user.

The second attachment portions 12a, 12b are configured in use to extend around at least a portion of the lower leg attachment portion 2.

Each second attachment portion 12a, 12b has a first end 13a, 13b located in use at or adjacent the lower leg attachment portion 2, and a second opposed end 14a, 14b comprising a shoe attachment member 15, in the form of a hook, configured in use for direct engagement with a fastener of a shoe of a user.

In the illustrated embodiment, the pair of second attachment portions 12a, 12b are provided by a single elongate member such that the first ends 13a, 13b are joined together and the second ends 14a, 14b are joined together. It is however to be understood that in some embodiments, the pair of second attachment portions may be provided by separate elongate members which are not joined at the first and/or second ends.

Each second attachment portion 12a, 12b is received within and extends through the open ended channel of a corresponding guide portion 11a, 11b.

The device 1 further comprises an adjustment mechanism 16 in the form of a toggle configured in use to adjust the separation of the shoe attachment member 15 at the second opposed end 14a, 14b of the second attachment portion 12a, 12b from the lower leg attachment portion 2.

The adjustment mechanism 16 is located at or adjacent the first ends 13a, 13b of the second attachment portion 12a, 12b.

In use, the user positions the lower leg attachment portion 2 (in the first open position) such that the inner surface 5 contacts the skin of the lower leg of the user.

The lower leg attachment portion 2 may be provided, in the first open position, such that the first end 3 of the lower leg attachment portion is spaced apart, disengaged and separated from the second opposed end 4 thereof.

The user may single handedly insert the first end 3 of the lower leg member 2 through aperture 10 adjacent the second end 4 thereof to form a looped portion to surround the lower leg of a user.

Alternatively, the lower leg attachment portion 2 may be provided, in the first open position, in a loop form, such that the first end 3 of the lower leg attachment portion 2 extends through the aperture at the second end 4 thereof, and is disengaged from and moveable, in a circumferential direction, relative to the second end 4.

The user may insert a lower leg into the opening formed by the loop provided by the lower leg attachment portion 2. The user may then move the first end 3 of the lower leg attachment portion 2, in a circumferential direction, relative to the second end 4 to tighten the lower leg attachment portion 2 against the lower leg in order to retain the attachment portion 2 is a desired location adjacent the lower leg. The first end 3 is then folded back across an adjacent portion of the lower leg attachment portion 2 (for example in an opposed circumferential direction of the loop formed) such that the first attachment member 8 is aligned with the second attachment member 9 for cooperative releasable engagement to provide the lower leg attachment portion 2 in the second closed position.

The user then engages the shoe attachment member 15, from the outside of the shoe, with the fastener of the shoe. The user can then adjust the adjustment mechanism 16 to tighten the second attachment portions 12a, 12b as necessary to provide the required support.

The ankle-foot orthopaedic device of the present invention is able to be independently put onto the lower leg of a user using a single handed operation ensuring that the user is able to act independently. Conventional devices including rigid ankle foot orthosis (AFO) and elasticated braces are very difficult to put on and require the user to have additional assistance when doing so.

Furthermore, the ankle-foot orthopaedic device of the present invention is adjustable so that the device can expand as the user (for example child) grows or to accommodate foot and ankle swelling. This ensures that one orthopaedic device can be used repeatedly requiring fewer replacements.

The ankle-foot orthopaedic device of the present invention only covers a small region of the lower leg of a user and does not contact the foot of the user. This provides increased comfort and compliance by the user.

The lower leg attachment portion is preferably moveable between:
a first open position in which the first end of the lower leg attachment portion is disengaged from the second opposed end thereof; and
a second closed position in which a portion of the lower leg attachment portion adjacent the first end thereof extends through the aperture at the second end, and the at least one attachment member secures the first end adjacent an outer surface of the lower leg attachment portion.

According to a second aspect, the present invention provides a method of single-handed installation of an ankle-foot orthopaedic device as claimed in any preceding claim on to the leg and shoe of a user;
positioning an openable lower leg attachment portion to extend around a portion of a lower leg of a user;
optionally passing a first end of the lower leg attachment portion through the aperture provided at a second end thereof is engageable to a second end thereof;
adjusting the position of the first end relative to the second end of the lower leg attachment portion to position the lower leg attachment portion adjacent the lower leg of a user;
securing the first end to a portion of the lower leg attachment portion; and
engaging the shoe attachment member with a fastener of a shoe of a user.

The ankle-foot orthopaedic device is preferably a child's ankle-foot orthopaedic device. It is however to be understood that the ankle-foot orthopaedic device may be used by any suitable user and is not to be considered to be limited for use by a child.

The ankle-orthopaedic device is preferably able to be put on by a single hand of a user. As a result, the ankle-orthopaedic device may be able to be used by young children who may also suffer from weakness in one arm. Furthermore, the ankle-orthopaedic device of the present invention therefore enables the user to put the splint on independently without requiring assistance from others. The ankle-orthopaedic device of the present invention therefore encourages the user to develop an independent life without impacting on the physical, emotional and social aspects of the user's life.

The ankle-foot orthopaedic device is preferably free from contact with a foot or inner portion of a shoe of a user. As a result, the device of the present invention does not have any engagement with the inside of a user's shoe. The user may therefore wear the device without the device creating an obstruction preventing the user's normal shoes to be worn.

The shoe attachment member is preferably releasably engageable to the fastener of a shoe of a user. The shoe attachment member is preferably releasably engageable to the fastner of a shoe of a user from the outside of a shoe of a user. As such, the user may easily and effectively change shoes as required without having to dismantle the device or device/shoe combination. In contrast, the user may simply disengage the shoe attachment member from the fastener of the first shoe and re-engage the shoe attachment member with fastener of a second shoe. The ankle-foot orthopaedic device of the present invention therefore enables the user to have a greater degree of freedom with regards to changing footwear.

## Claims

1. An ankle-foot orthopaedic device (1) for the treatment of foot drop, the device (1) comprising:
an openable lower leg attachment portion (2) configured in use to be positioned against and to extend around a portion of a lower leg of a user, in which a first end (3) of the lower leg attachment portion (2) is engageable to a portion of the lower leg attachment portion (2) at or adjacent a second end thereof (4), in which the second end (4) provides an aperture (10) configured to enable the first end (3) to pass therethrough, and in which the first end (3) of the lower leg attachment portion (2) comprises at least one attachment member (8) for securing the first end (3) in position relative to the second end (4);
a second attachment portion (12a, 12b) comprising an elongate member having a first end (13a, 13b) located at or adjacent the openable lower leg attachment portion (2), and a second opposed end (14a, 14b) comprising a shoe attachment member (15) configured in use for direct engagement with a fastener of a shoe of a user, in which the shoe attachment member comprises a hooked portion,
and in which the lower leg attachment portion (2) further comprises a pair of guide members (11a, 11b) provided on opposing sides of the lower leg attachment portion, each guide member (11a, 11b) defining an open-ended channel configured in use to receive at least a portion of the second attachment portion (12a, 12b) therein and to enable movement of the second attachment portion (12a, 12b) through the channel,
**characterized in that** the lower leg attachment portion is T-shaped such that the first end of the lower leg attachment portion has a smaller width than the second end of the lower leg attachment portion, and in which the aperture at or adjacent the second end of the lower leg attachment portion is sized to enable to receive the full width of the first end of the lower leg attachment portion.

2. An ankle-foot orthopaedic device (1) as claimed in claim 1, in which the lower leg attachment portion (2) is an elongate member.

3. An ankle-foot orthopaedic device (1) as claimed in either of claims 1 and 2, in which the lower leg attachment portion (2) comprises an inner surface (5) configured in use to be positioned adjacent a lower leg of a user, and an opposed outer surface (6), and in which the outer surface (6) provides the at least one attachment member (9), and in which the lower leg attachment portion (2) is moveable between:
a first open position in which the first end (3) of the lower leg attachment portion (2) is disengaged from the second opposed end thereof (4); and
a second closed position in which a portion of the lower leg attachment portion (2) adjacent the first end (3) thereof extends through the aperture (10) at the second end (4), and the at least one attachment member (8) secures the first end (3) adjacent an outer surface (6) of the lower leg attachment portion (2),
optionally in which the lower leg attachment portion (2) comprises at least one pair of cooperative attachment members (8, 9), a first attachment member (8) being located on the outer surface (6) of the lower leg attachment portion (2) at or adjacent the first end (3) thereof and a cooperative second attachment member (9) being located on the outer surface (6) of the lower leg attachment portion (2) and spaced apart from the first attachment member (8).

4. An ankle-foot orthopaedic device (1) as claimed in any one of claims 1 to 3, in which the at least one lower leg attachment member (2) comprises hook and loop fasteners.

5. An ankle-foot orthopaedic device (1) as claimed in any one of claims 1 to 4, in which the elongate member is a tubular member.

6. An ankle-foot orthopaedic device (1) as claimed in any preceding claim1, further comprising an adjustment mechanism (16) configured in use to adjust the separation of the shoe attachment member (15) at the second opposed end (14a, 14b) of the second attachment portion (12a, 12b) from the lower leg attachment portion (2), optionally in which the adjustment mechanism (16) comprises a toggle mechanism in communication with the elongate member to control the extension of the second opposed end (14a, 14b) of the elongate member from the lower leg attachment portion (2) .

7. An ankle-foot orthopaedic device (1) as claimed in any preceding claim, in which at least a portion of the second attachment portion (12a, 12b) is configured in use to extend around at least a portion of the lower leg attachment portion (2), optionally in which the second attachment portion (12a, 12b) is configured in use to extend entirely around the lower leg attachment portion (2).

8. An ankle-foot orthopaedic device (1) as claimed in any preceding claim, in which the at least one guide member (16) is provided on an outer surface (6) of the lower leg attachment portion (2).

9. An ankle-foot orthopaedic device (1) as claimed in any preceding claim, in which the device (1) is free from contact with a foot of a user.

10. An ankle-foot orthopaedic device (1) as claimed in any preceding claim, in which the lower leg attachment portion (2) is composed of flexible material, optionally in which the lower leg attachment portion (2) is composed of neoprene.

11. An ankle-foot orthopaedic device (1) as claimed in any preceding claim, in which the lower leg attachment portion (2) comprises an elasticated portion.

12. A method of single-handed installation of an ankle-foot orthopaedic device (1) as claimed in any preceding claim on to the leg and shoe of a user;
**characterized by** positioning an openable lower leg attachment portion (2) to extend around a portion of a lower leg of a user;
passing a first end (3) of the lower leg attachment portion (2) through the aperture (10) provided at a second end (4) thereof to be engageable to a second end (4) thereof;
adjusting the position of the first end (3) relative to the second end (4) of the lower leg attachment portion (2) to position the lower leg attachment portion (2) adjacent the lower leg of a user;
securing the first end (3) to a portion of the lower leg attachment portion (2) at or adjacent a second end (4) thereof; and
engaging the shoe attachment member (15) with a fastener of a shoe of a user.

13. A method as claimed in claim 12, further comprising using an adjustment mechanism (16) to adjust the separation of the shoe attachment member (15) at the second opposed end of the second attachment portion (12a, 12b) from the lower leg attachment portion (2).

14. A method for manufacturing an ankle-foot orthopaedic device (1) as claimed in claim 1, **characterized by** the method comprising:
obtaining an openable lower leg attachment portion (2) configured in use to be positioned against and to extend around a portion of a lower leg of a user, in which a first end (3) of the lower leg attachment portion (2) is engageable to a portion of the lower leg attachment portion (2) at or adjacent a second opposed end (4) thereof, in which the second end (4) provides an aperture (10) configured to enable the first end (3) to pass therethrough, and in which the first end (3) of the lower leg attachment portion (2) comprises at least one attachment member (8) for securing the first end (3) in position relative to the second end (4); in which the lower leg attachment portion (2) further comprises a pair of guide members (11a, 11b) provided on opposing sides of the lower leg attachment portion, each guide member (11a, 11b) defining an open-ended channel configured in use to receive at least a portion of the second attachment portion (!2a, 12b) therein and to enable movement of the second attachment portion (!2a, 12b) through the channel; and in which the lower leg attachment portion is T-shaped such that the first end of the lower leg attachment portion has a smaller width than the second end of the lower leg attachment portion, and in which the aperture at or adjacent the second end of the lower leg attachment portion is sized to enable to receive the full width of the first end of the lower leg attachment portion;
obtaining a second attachment portion (12a, 12b) comprising an elongate member having a first end (13a, 13b), and a second opposed end (!4a, 14b) comprising a shoe attachment member (15) configured in use for direct engagement with a fastener of a shoe of a user, in which the shoe attachment member comprises a hooked portion,
and
engaging a first end (13a, 13b) of a second attachment portion (12a, 12b) comprising an elongate member at or adjacent the openable lower leg attachment portion (2); and
arranging the second attachment portion (12a, 12b) to extend through the channels of each guide portion (11a, 11b).

## Patentansprüche

1. Orthopädische Vorrichtung für Knöchel und Fuß (1) zur Behandlung von Fußabfall, wobei die Vorrichtung (1) Folgendes umfasst:
einen öffenbaren Unterschenkelanbringungsabschnitt (2), der bei Verwendung dazu konfiguriert ist, gegen einen Abschnitt eines Unterschenkels eines Benutzers positioniert zu sein und sich darum zu erstrecken, wobei ein erstes Ende (3) des Unterschenkelanbringungsabschnittes (2) mit einem Abschnitt des Unterschenkelanbringungsabschnittes (2) an oder benachbart zu einem zweiten Ende davon (4) in Eingriff bringbar ist, wobei das zweite Ende (4) einen Durchgang (10) bereitstellt, der dazu konfiguriert ist, dem ersten Ende (3) zu ermöglichen, dort hindurch zu verlaufen, und wobei das erste Ende (3) des Unterschenkelanbringungsabschnittes (2) zumindest ein Anbringungselement (8) zum Sichern des ersten Endes (3) in Position relativ zu dem zweiten Ende (4) umfasst;
einen zweiten Anbringungsabschnitt (12a, 12b), der ein längliches Element mit einem ersten Ende (13a, 13b), das sich an oder benachbart zu dem öffenbaren Unterschenkelanbringungsabschnitt (2) befindet, und ein zweites gegenüberliegendes Ende (14a, 14b) umfasst, das ein Schuhanbringungselement (15) umfasst, das bei Verwendung zum direkten Eingriff mit einer Befestigung eines Schuhs eines Benutzers konfiguriert ist, wobei das Schuhanbringungselement einen Hakenabschnitt umfasst,
und wobei der Unterschenkelanbringungsabschnitt (2) ferner ein Paar Führungselemente (11a, 11b) umfasst, das an gegenüberliegenden Seiten des Unterschenkelanbringungsabschnittes bereitgestellt ist, wobei jedes Führungselement (11a, 11b) einen Kanal mit offenem Ende definiert, der bei Verwendung dazu konfiguriert ist, zumindest einen Abschnitt des zweiten Anbringungsabschnittes (12a, 12b) darin aufzunehmen und Bewegung des zweiten Anbringungsabschnittes (12a, 12b) durch den Kanal zu ermöglichen,
**dadurch gekennzeichnet, dass** der Unterschenkelanbringungsabschnitt T-förmig ist, sodass das erste Ende des Unterschenkelanbringungsabschnittes eine kleinere Breite als das zweite Ende des Unterschenkelanbringungsabschnittes aufweist, und wobei der Durchgang an oder benachbart zu dem zweiten Ende des Unterschenkelanbringungsabschnittes bemessen ist, um zu ermöglichen, die volle Breite des ersten Endes des Unterschenkelanbringungsabschnittes aufzunehmen.

2. Orthopädische Vorrichtung für Knöchel und Fuß (1) nach Anspruch 1, wobei der Unterschenkelanbringungsabschnitt (2) ein längliches Element ist.

3. Orthopädische Vorrichtung für Knöchel und Fuß (1) nach Anspruch 1 und 2, wobei der Unterschenkelanbringungsabschnitt (2) eine Innenfläche (5), die bei Verwendung dazu konfiguriert ist, benachbart zu einem Unterschenkel eines Benutzers positioniert zu sein, und eine gegenüberliegende Außenfläche (6) umfasst, und wobei die Außenfläche (6) das zumindest eine Anbringungselement (9) bereitstellt, und wobei der Unterschenkelanbringungsabschnitt (2) beweglich ist zwischen:
einer ersten offenen Position, wobei das erste Ende (3) des Unterschenkelanbringungsabschnittes (2) von dem zweiten gegenüberliegenden Ende davon (4) gelöst ist; und
einer zweiten geschlossenen Position, wobei sich ein Abschnitt des Unterschenkelanbringungsabschnittes (2) benachbart zu dem erstem Ende (3) davon durch den Durchgang (10) an dem zweiten Ende (4) erstreckt und das zumindest eine Anbringungselement (8) das erste Ende (3) benachbart zu einer Außenfläche (6) des Unterschenkelanbringungsabschnittes (2) sichert,
wobei optional der Unterschenkelanbringungsabschnitt (2) zumindest ein Paar kooperierender Anbringungselemente (8, 9) umfasst, wobei sich ein erstes Anbringungselement (8) an der Außenfläche (6) des Unterschenkelanbringungsabschnittes (2) an oder benachbart zu dem ersten Ende (3) davon befindet und sich ein kooperierendes zweites Anbringungselement (9) an der Außenfläche (6) des Unterschenkelanbringungsabschnittes (2) und beabstandet von dem ersten Anbringungselement (8) befindet.

4. Orthopädische Vorrichtung für Knöchel und Fuß (1) nach einem der Ansprüche 1 bis 3, wobei das zumindest eine Unterschenkelanbringungselement (2) Klettverschlussbefestigungen umfasst.

5. Orthopädische Vorrichtung für Knöchel und Fuß (1) nach einem der Ansprüche 1 bis 4, wobei das längliche Element ein rohrförmiges Element ist.

6. Orthopädische Vorrichtung für Knöchel und Fuß (1) nach einem vorhergehenden Anspruch, ferner umfassend einen Einstellmechanismus (16), der bei Verwendung dazu konfiguriert ist, die Trennung des Schuhanbringungselements (15) an dem zweiten gegenüberliegenden Ende (14a, 14b) des zweiten Anbringungsabschnittes (12a, 12b) von dem Unterschenkelanbringungsabschnitt (2) einzustellen, wobei optional der Einstellmechanismus (16) einen Kippmechanismus in Kommunikation mit dem länglichen Element umfasst, um die Erstreckung des zweiten gegenüberliegenden Endes (14a, 14b) des länglichen Elements von dem Unterschenkelanbringungsabschnitt (2) zu steuern.

7. Orthopädische Vorrichtung für Knöchel und Fuß (1) nach einem vorhergehenden Anspruch, wobei zumindest ein Abschnitt des zweiten Anbringungsabschnittes (12a, 12b) bei Verwendung dazu konfiguriert ist, sich um zumindest einen Abschnitt des Unterschenkelanbringungsabschnittes (2) zu erstrecken, wobei optional der zweite Anbringungsabschnitt (12a, 12b) bei Verwendung dazu konfiguriert ist, sich vollständig um den Unterschenkelanbringungsabschnitt (2) zu erstrecken.

8. Orthopädische Vorrichtung für Knöchel und Fuß (1) nach einem vorhergehenden Anspruch, wobei das zumindest eine Führungselement (16) an einer Außenfläche (6) des Unterschenkelanbringungsabschnittes (2) bereitgestellt ist.

9. Orthopädische Vorrichtung für Knöchel und Fuß (1) nach einem vorhergehenden Anspruch, wobei die Vorrichtung (1) frei von Kontakt mit einem Fuß eines Benutzers ist.

10. Orthopädische Vorrichtung für Knöchel und Fuß (1) nach einem vorhergehenden Anspruch, wobei der Unterschenkelanbringungsabschnitt (2) aus flexiblem Material besteht, wobei optional der Unterschenkelanbringungsabschnitt (2) aus Neopren besteht.

11. Orthopädische Vorrichtung für Knöchel und Fuß (1) nach einem vorhergehenden Anspruch, wobei der Unterschenkelanbringungsabschnitt (2) einen elastischen Abschnitt umfasst.

12. Verfahren zur einhändigen Installation einer orthopädischen Vorrichtung für Knöchel und Fuß (1) nach einem vorhergehenden Anspruch an dem Bein und Schuh eines Benutzers;
**gekennzeichnet durch**
Positionieren eines öffenbaren Unterschenkelanbringungsabschnittes (2), um sich um einen Abschnitt eines Unterschenkels eines Benutzers zu erstrecken;
Verlaufenlassen eines ersten Endes (3) des Unterschenkelanbringungsabschnittes (2) durch den Durchgang (10), der an einem zweiten Ende (4) davon bereitgestellt ist, um mit einem zweiten Ende (4) davon in Eingriff bringbar zu sein;
Einstellen der Position des ersten Endes (3) relativ zu dem zweiten Ende (4) des Unterschenkelanbringungsabschnittes (2), um den Unterschenkelanbringungsabschnitt (2) benachbart zu dem Unterschenkel eines Benutzers zu positionieren;
Sichern des ersten Endes (3) an einem Abschnitt des Unterschenkelanbringungsabschnittes (2) an oder benachbart zu einem zweiten Ende (4) davon; und
Ineingriffbringen des Schuhanbringungselements (15) mit einer Befestigung eines Schuhs eines Benutzers.

13. Verfahren nach Anspruch 12, ferner umfassend Verwenden eines Einstellmechanismus (16), um die Trennung des Schuhanbringungselements (15) an dem zweiten gegenüberliegenden Ende des zweiten Anbringungsabschnittes (12a, 12b) von dem Unterschenkelanbringungsabschnitt (2) einzustellen.

14. Verfahren zum Herstellen einer orthopädischen Vorrichtung für Knöchel und Fuß (1) nach Anspruch 1, **dadurch gekennzeichnet, dass** das Verfahren Folgendes umfasst:
Erhalten eines öffenbaren Unterschenkelanbringungsabschnittes (2), der bei Verwendung dazu konfiguriert ist, gegen einen Abschnitt eines Unterschenkels eines Benutzers positioniert zu sein und sich darum zu erstrecken, wobei ein erstes Ende (3) des Unterschenkelanbringungsabschnittes (2) an einem Abschnitt des Unterschenkelanbringungsabschnittes (2) an oder benachbart zu einem zweiten gegenüberliegenden Ende (4) davon in Eingriff bringbar ist, wobei das zweite Ende (4) einen Durchgang (10) bereitstellt, der dazu konfiguriert ist, dem ersten Ende (3) zu ermöglichen, dort hindurch zu verlaufen, und wobei das erste Ende (3) des Unterschenkelanbringungsabschnittes (2) zumindest ein Anbringungselement (8) zum Sichern des ersten Endes (3) in Position relativ zu dem zweiten Ende (4) umfasst; wobei der Unterschenkelanbringungsabschnitt (2) ferner ein Paar Führungselemente (11a, 11b) umfasst, das auf gegenüberliegenden Seiten des Unterschenkelanbringungsabschnittes bereitgestellt ist, wobei jedes Führungselement (11a, 11b) einen Kanal mit offenem Ende definiert, der bei Verwendung dazu konfiguriert ist, zumindest einen Abschnitt des zweiten Anbringungsabschnittes (12a, 12b) darin aufzunehmen und Bewegung des zweiten Anbringungsabschnittes (12a, 12b) durch den Kanal zu ermöglichen; und wobei der Unterschenkelanbringungsabschnitt T-förmig ist, sodass das erste Ende des Unterschenkelanbringungsabschnittes eine kleinere Breite als das zweite Ende des Unterschenkelanbringungsabschnittes aufweist, und wobei der Durchgang an oder benachbart zu dem zweiten Ende des Unterschenkelanbringungsabschnittes bemessen ist, um zu ermöglichen, die volle Breite des ersten Endes des Unterschenkelanbringungsabschnittes aufzunehmen;
Erhalten eines zweiten Anbringungsabschnittes (12a, 12b), der ein längliches Element mit einem ersten Ende (13a, 13b) und einem zweiten gegenüberliegenden Ende (14a, 14b) umfasst, das ein Schuhanbringungselement (15) umfasst, das bei Verwendung für direkten Eingriff mit einer Befestigung eines Schuhs eines Benutzers konfiguriert ist, wobei das Schuhanbringungselement einen Hakenabschnitt umfasst,
und
Eingreifen in ein erstes Ende (13a, 13b) eines zweiten Anbringungsabschnittes (12a, 12b), der ein längliches Element umfasst, an oder benachbart zu dem öffenbaren Unterschenkelanbringungsabschnitt (2); und
Anordnen des zweiten Anbringungsabschnittes (12a, 12b), um sich durch die Kanäle jedes Führungsabschnittes (11a, 11b) zu erstrecken.

## Revendications

1. Dispositif orthopédique cheville-pied (1) pour le traitement du pied tombant, le dispositif (1) comprenant :
une partie de fixation de jambe inférieure pouvant être ouverte (2) conçue, lors de l'utilisation, pour se positionner contre une partie d'une jambe inférieure d'un utilisateur et s'étendre autour de celle-ci, dans laquelle une première extrémité (3) de la partie de fixation de jambe inférieure (2) peut être mise en prise avec une partie de la partie de fixation de jambe inférieure (2) au niveau ou à proximité d'une seconde extrémité (4) de celle-ci, dans laquelle la seconde extrémité (4) fournit une ouverture (10) conçue pour permettre à la première extrémité (3) de la traverser, et dans laquelle la première extrémité (3) de la partie de fixation de jambe inférieure (2) comprend au moins un élément de fixation (8) destiné à fixer la première extrémité (3) en position par rapport à la seconde extrémité (4) ;
une seconde partie de fixation (12a, 12b) comprenant un élément allongé possédant une première extrémité (13a, 13b) située au niveau ou à proximité de la partie de fixation inférieure de jambe pouvant être ouverte (2), et une seconde extrémité opposée (14a, 14b) comprenant un élément de fixation de chaussure (15) conçu, lors de l'utilisation, pour une mise en prise directe avec une attache d'une chaussure d'un utilisateur, dans laquelle l'élément de fixation de chaussure comprend une partie en forme de crochet,
et dans lequel la partie de fixation de jambe inférieure (2) comprend en outre une paire d'éléments de guidage (11a, 11b) prévus sur les côtés opposés de la partie de fixation de jambe inférieure, chaque élément de guidage (11a, 11b) définissant un canal à extrémité ouverte conçu, lors de l'utilisation, pour recevoir au moins une partie de la seconde partie de fixation (12a, 12b) en son sein et pour permettre le déplacement de la seconde partie de fixation (12a, 12b) à travers le canal,
**caractérisé en ce que** la partie de fixation de jambe inférieure est en forme de T de sorte que la première extrémité de la partie de fixation de jambe inférieure présente une largeur plus petite que la seconde extrémité de la partie de fixation de jambe inférieure, et dans lequel l'ouverture au niveau ou à proximité de la seconde extrémité de la partie de fixation de jambe inférieure est dimensionnée de manière à permettre la réception de toute la largeur de la première extrémité de la partie de fixation de jambe inférieure.

2. Dispositif orthopédique cheville-pied (1) selon la revendication 1, ladite partie de fixation de jambe inférieure (2) étant un élément allongé.

3. Dispositif orthopédique cheville-pied (1) selon l'une quelconque des revendications 1 et 2, dans lequel la partie de fixation de jambe inférieure (2) comprend une surface interne (5) conçue, lors de l'utilisation, pour être positionnée à proximité d'une jambe inférieure d'un utilisateur, et une surface externe opposée (6), et dans lequel la surface externe (6) fournit ledit au moins un élément de fixation (9), et dans lequel la partie de fixation de jambe inférieure (2) est mobile entre :
une première position ouverte dans laquelle la première extrémité (3) de la partie de fixation de jambe inférieure (2) est désaccouplée de la seconde extrémité opposée (4) de celle-ci ; et
une seconde position fermée dans laquelle une partie de la partie de fixation de jambe inférieure (2) à proximité de la première extrémité (3) de celle-ci s'étend à travers l'ouverture (10) au niveau de la seconde extrémité (4), et ledit au moins un élément de fixation (8) fixe la première extrémité (3) à proximité d'une surface externe (6) de la partie de fixation de jambe inférieure (2),
éventuellement dans lequel la partie de fixation de jambe inférieure (2) comprend au moins une paire d'éléments de fixation coopératifs (8, 9), un premier élément de fixation (8) étant situé sur la surface externe (6) de la partie de fixation de jambe inférieure (2) au niveau ou à proximité de la première extrémité (3) de celle-ci et un second élément de fixation coopératif (9) étant situé sur la surface externe (6) de la partie de fixation de jambe inférieure (2) et espacé du premier élément de fixation (8).

4. Dispositif orthopédique cheville-pied (1) selon l'une quelconque des revendications 1 à 3, dans lequel ledit au moins un élément de fixation de jambe inférieure (2) comprend des attaches à crochets et boucles.

5. Dispositif orthopédique cheville-pied (1) selon l'une quelconque des revendications 1 à 4, dans lequel l'élément allongé est un élément tubulaire.

6. Dispositif orthopédique cheville-pied (1) selon l'une quelconque des revendications précédentes, comprenant en outre un mécanisme de réglage (16) conçu, lors de l'utilisation, pour régler la séparation de l'élément de fixation de chaussure (15) au niveau de la seconde extrémité opposée (14a, 14b) de la seconde partie de fixation (12a, 12b) par rapport à la partie de fixation de jambe inférieure (2), éventuellement dans lequel le mécanisme de réglage (16) comprend un mécanisme à bascule en communication avec l'élément allongé de manière à commander l'extension de la seconde extrémité opposée (14a, 14b) de l'élément allongé par rapport à la partie de fixation de jambe inférieure (2).

7. Dispositif orthopédique cheville-pied (1) selon l'une quelconque des revendications précédentes, dans lequel au moins une partie de la seconde partie de fixation (12a, 12b) est conçue, lors de l'utilisation, pour s'étendre autour d'au moins une partie de la partie de fixation de jambe inférieure (2), éventuellement dans lequel la seconde partie de fixation (12a, 12b) est conçue, lors de l'utilisation, pour s'étendre entièrement autour de la partie de fixation de jambe inférieure (2).

8. Dispositif orthopédique cheville-pied (1) selon l'une quelconque des revendications précédentes, dans lequel ledit au moins un élément de guidage (16) est prévu sur une surface externe (6) de la partie de fixation de jambe inférieure (2).

9. Dispositif orthopédique cheville-pied (1) selon l'une quelconque des revendications précédentes, ledit dispositif (1) étant libre de tout contact avec le pied d'un utilisateur.

10. Dispositif orthopédique cheville-pied (1) selon l'une quelconque des revendications précédentes, dans lequel la partie de fixation de jambe inférieure (2) est composée d'un matériau flexible, éventuellement dans lequel la partie de fixation de jambe inférieure (2) est composée de néoprène.

11. Dispositif orthopédique cheville-pied (1) selon l'une quelconque des revendications précédentes, dans lequel la partie de fixation de jambe inférieure (2) comprend une partie élastique.

12. Procédé d'installation d'une seule main d'un dispositif orthopédique cheville-pied (1) selon l'une quelconque des revendications précédentes sur la jambe et la chaussure d'un utilisateur ;
**caractérisé par** le positionnement d'une partie de fixation de jambe inférieure pouvant être ouverte (2) de manière à s'étendre autour d'une partie d'une jambe inférieure d'un utilisateur ;
le passage d'une première extrémité (3) de la partie de fixation de jambe inférieure (2) à travers l'ouverture (10) pourvue au niveau d'une seconde extrémité (4) de celle-ci de manière à pouvoir être mise en prise avec une seconde extrémité (4) de celle-ci ;
le réglage de la position de la première extrémité (3) par rapport à la seconde extrémité (4) de la partie de fixation de jambe inférieure (2) de manière à positionner la partie de fixation de jambe inférieure (2) à proximité de la jambe inférieure d'un utilisateur ;
la fixation de la première extrémité (3) à une partie de la partie de fixation de jambe inférieure (2) au niveau ou à proximité d'une seconde extrémité (4) de celle-ci ; et
la mise en prise de l'élément de fixation de chaussure (15) avec une attache d'une chaussure d'un utilisateur.

13. Procédé selon la revendication 12, comprenant en outre l'utilisation d'un mécanisme de réglage (16) pour régler la séparation de l'élément de fixation de chaussure (15) au niveau de la seconde extrémité opposée de la seconde partie de fixation (12a, 12b) par rapport à la partie de fixation de jambe inférieure (2).

14. Procédé permettant la fabrication d'un dispositif orthopédique cheville-pied (1) selon la revendication 1, **caractérisé en ce que** le procédé comprend :
l'obtention d'une partie de fixation de jambe inférieure pouvant être ouverte (2) conçue, lors de l'utilisation, pour se positionner contre une partie d'une jambe inférieure d'un utilisateur et s'étendre autour de celle-ci, dans laquelle une première extrémité (3) de la partie de fixation de jambe inférieure (2) peut être mise en prise avec une partie de la partie de fixation de jambe inférieure (2) au niveau ou à proximité d'une seconde extrémité opposée (4) de celle-ci, dans laquelle la seconde extrémité (4) fournit une ouverture (10) conçue pour permettre à la première extrémité (3) de la traverser, et dans laquelle la première extrémité (3) de la partie de fixation de jambe inférieure (2) comprend au moins un élément de fixation (8) destiné à fixer la première extrémité (3) en position par rapport à la seconde extrémité (4) ; dans laquelle la partie de fixation de jambe inférieure (2) comprend en outre une paire d'éléments de guidage (11a, 11b) pourvus sur les côtés opposés de la partie de fixation de jambe inférieure, chaque élément de guidage (11a, 11b) définissant un canal à extrémité ouverte conçu, lors de l'utilisation, pour recevoir au moins une partie de la seconde partie de fixation (12a, 12b) en son sein et pour permettre le déplacement de la seconde partie de fixation (12a, 12b) à travers le canal ; et dans laquelle la partie de fixation de jambe inférieure est en forme de T de sorte que la première extrémité de la partie de fixation de jambe inférieure présente une largeur plus petite que la seconde extrémité de la partie de fixation de jambe inférieure, et dans laquelle l'ouverture au niveau ou à proximité de la seconde extrémité de la partie de fixation de jambe inférieure est dimensionnée de manière à permettre la réception de toute la largeur de la première extrémité de la partie de fixation de jambe inférieure ;
l'obtention d'une seconde partie de fixation (12a, 12b) comprenant un élément allongé possédant une première extrémité (13a, 13b) et une seconde extrémité opposée (14a, 14b) comprenant un élément de fixation de chaussure (15) conçu, lors de l'utilisation, pour une mise en prise directe avec une attache d'une chaussure d'un utilisateur, dans laquelle l'élément de fixation de chaussure comprend une partie en forme de crochet,
et
la mise en prise d'une première extrémité (13a, 13b) d'une seconde partie de fixation (12a, 12b) comprenant un élément allongé au niveau ou à proximité de la partie de fixation inférieure de jambe pouvant être ouverte (2) ; et
l'agencement de la seconde partie de fixation (12a, 12b) pour qu'elle s'étende à travers les canaux de chaque partie de guidage (11a, 11b).
